# EUROPÄISCHE PATENTSCHRIFT

(11) **EP 2 869 802 B1**
(45) Veröffentlichungstag und Bekanntmachung des Hinweises auf die Patenterteilung: **18.05.2016**
(21) Anmeldenummer: 13734750.6
(22) Anmeldetag: 05.07.2013
(51) Int. Cl.: A61F 13/20

(54) **TAMPON FÜR ZWISCHENMENSTRUATIONSTAGE**
TAMPON FOR DAYS BETWEEN MENSTRUATIONS
TAMPON POUR LES PÉRIODES INTERMENSTRUELLES

(30) Priorität: 06.07.2012 AT 7512012
(43) Veröffentlichungstag der Anmeldung: 13.05.2015
(73) Patentinhaber: Ruggli Projects AG, 6332 Hagendorn (CH)
(72) Erfinder: MÜLLER, Peter, CH-8004 Zürich (CH)
(74) Vertreter: Burger, Hannes
(86) Internationale Anmeldenummer: PCT/EP2013/064277
(87) Internationale Veröffentlichungsnummer: WO 2014/006188

(56) Entgegenhaltungen:
- WO-A2-02/17844
- US-B1- 7 014 637

## Beschreibung

Die Erfindung betrifft einen Tampon für Zwischenmenstruationstage der zumindest einen Saugkörper, von dem zumindest ein Teil seines Volumens aus einem saugfähigen Material gebildet ist und einen Mittelteil, ein proximales Ende und ein distales Ende aufweist und einem mit dem Tampon verbundenen Auszugmittel..

Bisher wurden Tampons während der Menstruation anstelle von Binden verwendet. Nachteilig ist bei diesen Binden, dass Frauen oft empfindlich oder sogar allergisch auf eine Binde reagieren oder die Binde als unangenehm empfinden.

Die im Stand der Technik bekannten herkömmlichen Tampons werden insbesondere als Menstruationstampon verwendet und über eine Körperöffnung, insbesondere die Vagina, eingeführt, wobei der Tampon in dieser Körperöffnung platziert werden kann. Derartige Tampons sind beispielsweise aus der EP 0 422 660, EP 0 611 562, US 2,499,414, DE 60 2004 003 888, DE 1 9825 877A, DE 37 39 163A, DE 4 304 505A, CH 248 634A, DE 1187 345A, DE 20 320 992A, DE 10 306 678A, DE 20 2008 020 640A, DE 20 2006 0007A, DE 20 2009 000 983U bekannt.

Im Stand der Technik sind folgende Abmessungen und Größen des herkömmlichen Tampons bekannt. Die Länge des herkömmlichen Tampons beträgt beispielsweise zwischen 40 mm bis 70 mm und Durchmesser ist größer 11 mm

Der herkömmliche Tampon kann aus saugfähigem Material, aus stark komprimiertem saugfähigen Material und/oder aus absorptionsfähigem Material bestehen. Er besitzt zum Entfernen aus der Körperöffnung ein Auszugsmittel z.B. einen Rückziehfaden.

Der herkömmliche Tampon kann Markierungen an seiner Oberfläche aufweisen, er kann in verschiedenen Farben hergestellt werden. Ebenfalls kann der Tampon mit einem chemischen Indikator hergestellt werden der die Farbe bei bestimmten Krankheiten verändert wie zum Beispiel bei Anämie, Diabetes, Hepatitis A,B oder C und HIV.

Auf Grund der Abmessungen des herkömmlichen Tampons wird dieser Tampon nicht jeden Tag verwendet, sondern nur an den Menstruationstagen und erfährt während des Tragens durch die in dieser Zeitperiode anfallende, hohe Flüssigkeitsmenge eine starke Gewichtserhöhung.

Aus der DE 1 491 169 C und der US 2 761 449 A sind Tampons bekannt bei welchen die Saugfähigkeit zwischen 4,4 und 6 g und zwischen 10,85 und 18,40 g liegt.

WO 0217844 offenbart einen Tampon, welcher mit einem Volumen des saugfähigen Materials zur Aufnahme von weniger als 6 g Flüssigkeitsmenge ausgebildet ist. US 7014637 offenbart Tampons dessen Saugvermögen durch einen Hohlraum verringert ist um toxischen Schock zu vermeiden.

Weiters sind aus der US 2849000A Tampons bekannt geworden, welche eine Saugfähigkeit von 6 bis 8 g aufweisen.

Des Weiteren ist es bekannt, für die Tage zwischen den Menstruationstagen zum Auffangen von austretenden Körperflüssigkeiten aus der Vagina, insbesondere auch während der Wechseljahre, Slipeinlagen zu verwenden. Diese führen jedoch bei vielen Frauen zu Beschwerden und Irritationen und sind daher in vielen Fällen nicht verwendbar.

Der vorliegenden Erfindung liegt daher die Aufgabe zu Grunde, einen Alltagstampon bzw. Tampon, der zwischen den einzelnen Menstruationsphasen angewendet werden kann, günstige Trageeigenschaften ermöglicht und bevorzugt preisgünstig herzustellen ist, zu schaffen.

Die Aufgabe wird erfindungsgemäss durch einen Tampon gemäß Anspruch 1 gelöst.

Die Vorteile eines derartigen Alltagstampon bzw. Tampon liegen darin, dass dieser Tampon unsichtbar getragen werden kann und zusätzlich geruchsvermindernd bzw. sogar geruchsverhindernd wirkt. Dazu kommt, dass dieser Tampon im Bereich des Vaginaeintritts zu keinen Irritationen führen kann. Vorteilhaft ist aber auch, dass dieser Tampon bei der sportlichen Betätigung, vor allem auch beim Schwimmen verwendbar ist und dadurch den Frauen auch eine hohe Unabhängigkeit bei der Bekleidung ermöglicht. Ein weiterer Vorteil liegt darin, dass die Muskulatur im Bereich der Scheide im Einführzustand des Tampons aber auch nach Aufnahme der Flüssigkeit nur eine geringe radiale und vertikale Belastung ausübt und damit ein hoher Tragekomfort erreicht wird, der auch eine tägliche Anwendung eines derartigen Tampons gestattet.

Der Tampn und/oder der Saugkörper weist eine Länge von kleiner 40 mm bevorzugt eine Länge zwischen 40 und 10 mm oder zwischen 38 und 30 mm auf, wodurch auch die Expansion und der Druck des Tampons gegen die Gebärmutter verringert und damit eine Abdichtung und ein Abdämmen der Austrittsöffnung der Gebärmutter, die erheblichen Diskomfort erzeugen kann, verhindert wird.

Weiters ist auch eine Ausgestaltung möglich, bei der der Saugkörper über sein gesamtes Volumen aus einem saugfähigen Material gebildet ist, wodurch bei einem Tampon die Abmessungen des Durchmessers und die Menge der aufnehmbaren optimiert werden können. Nach einer weiteren, vorteilhaften Weiterbildung ist vorgesehen, dass das saugfähige Material aus faserförmigem Material gebildet ist, wodurch die Tröpfchen der Flüssigkeit an den einzelnen Fasern anhaften können und damit eine hohe Absorptionsfähigkeit des Saugkörpers eines Alltagstampon bzw. Tampons erreicht wird.

Vorteilhaft ist aber auch, wenn das saugfähige Materials aus einem komprimierten, faserförmigen Material gebildet ist, da dadurch die Handhabung des Tampons, insbesondere das Einführen und Entnehmen des Tampons, erleichtert wird.

Das Volumen des saugfähigen Materials ist zur Aufnahme einer Flüssigkeitsmenge zwischen 0,5 g und 4 g bevorzugt 3,5 g ausgebildet.

Dadurch wird eine zu hohe Saugwirkung auf die Schleimhäute vermieden und auch die Entnahme des Alltagstampon bzw. Tampons bei geringem Flüssigkeitsaustritt erleichtert. Zudem kann auch das Risiko eines toxischen Schocksyndroms (TSS) leichter verhindert bzw. wesentlich vermindert werden.

Von Vorteil ist auch weiters, wenn ein maximaler Durchmesser des Saugkörpers bzw. eines den Saugkörper umschließenden Hüllkörpers maximal 10 mm beträgt, bevorzugt einen Wert zwischen 2 und 10 mm aufweist.. Durch diese geringen Durchmesser wird aber auch die Belastung der Muskulatur der Vagina verringert und ein besserer Tragekomfort erzielt. Zusätzlich wird durch den geringeren Durchmesser die Sphincter-Muskulatur der Blase geringer beansprucht.

Vorteilhaft ist es auch, wenn zumindest der Mittelteil des Saugkörpers oder ein diese umhüllender Hüllkörper zylinderförmig ausgebildet ist, wodurch eine gleichmäßige Anlage an den Schleimhäuten der Vagina erzielt werden kann.

Es kann sich aber auch als vorteilhaft erweisen, wenn zumindest der Mittelteil des Saugkörpers oder ein diesen umhüllender Hüllkörper kegel- bzw. kegelstumpfförmig ausgebildet ist, da das Einsetzen des Alltagstampon bzw. Tampons dadurch vereinfacht werden kann.

Nach einer anderen Weiterbildung ist vorgesehen, dass sich der Kegel bzw. der Kegelstumpf vom proximalen Ende bis zum distalen Ende durchgehend erstreckt, wodurch die Entnahme des Alltagstampon bzw. Tampons vereinfacht werden kann.

Weiters ist es aber auch möglich, dass zumindest innerhalb des Mittelteils des Saugkörpers ein Kernbereich aus faserförmigem Material angeordnet ist, welcher bei gleichem Volumen zur Aufnahme einer geringeren Flüssigkeitsmenge ausgebildet ist, als das Material des Saugkörpers. Dadurch kann trotz der gewünschten und bei unterschiedlichen anatomischen Ausbildungen benötigten größeren Durchmessern des Tampons die Saugwirkung bzw. das Ausmaß der Flüssigkeitsaufnahme vor allem im Saugkörper einfacher an die benötigten Ausmaße angepasst werden.

Weiters kann aber auch, zumindest innerhalb des Mittelteils des Saugkörpers, ein Kernbereich aus einem Material angeordnet sein, welches bei gleichem Volumen zur Aufnahme einer geringeren Flüssigkeitsmenge ausgebildet ist als das Material des Saugkörpers oder flüssigkeitsabweisend ist. Damit kann die aufzunehmende Flüssigkeitsmenge ausschließlich durch die Gestaltung und das Volumen des Saugkörpers festgelegt werden.

Es ist aber auch von Vorteil, wenn das faserförmige Material des Saugkörpers ein Gesamtgewicht von maximal 2 g, bevorzugt 1,7 g und ein minimales Gewicht von 0,5 g bevorzugt 0,2 g aufweist, da dadurch das Gewicht auch des mit Flüssigkeit gefüllten Tampons so gering ist, sodass die im vorstehenden Absatz erwähnten Erschwernisse verringert bzw. verhindert werden können.

Eine Anpassung der Saugwirkung und auch der Flüssigkeitsaufnahmefähigkeit des Tampons ist mit Vorteil dadurch möglich, dass der Saugkörper mit einem Hohlraum ausgebildet ist und im Bereich seines distalen Endes eine Öffnung zu diesem Hohlraum aufweist. Dadurch ist es möglich, einen einheitlichen Saugkörper herzustellen, der durch das Einsetzen von unterschiedlichen Kernbereichen mit unterschiedlicher dreidimensionaler Form insbesondere aber mit unterschiedlichem Durchmesser, unabhängig davon, ob diese über die gesamte Länge gleichbleibend oder wechselnd ist, unterschiedliche Außenabmessungen des Tampons zu erzielen und trotzdem die Aufnahmefähigkeit von Flüssigkeit und die dadurch bewirkte Saugwirkung gleichgehalten werden kann.

Weiters ist es vorteilhaft, wenn das Auszugmittel mit dem Saugkörper und/oder dem Kernbereich des Tampons verbunden ist, da dadurch eine sichere Entnahme des Tampons aus der Körperhöhle erzielt werden kann.

Nach einer anderen Weiterbildung ist vorgesehen, dass eine äußere Oberfläche des Saugkörpers bzw. der Mittelteil zumindest zum Teil mit einer Umhüllung, zum Beispiel einem Nonwoven, versehen ist. Dadurch wird ein Faserverlust und Festhaften von Fasern an den Schleimhäuten verringert bzw. ausgeschaltet.

Es ist aber auch möglich, dass nur das proximale Ende und der Mittelteil mit einer zumindest teilweise flüssigkeitsdurchlässigen Umhüllung, zum Beispiel einem Nonwoven, ausgebildet sind. Damit kann das Aufbringen des Nonwoven vereinfacht werden und wird vor allem in jenen Bereichen, in denen eine nahezu vollflächige Berührung mit der Schleimhaut auftritt, der Faserverlust vermieden.

Dabei ist es weiters vorteilhaft, wenn die Umhüllung durch ein Netz oder eine Lochfolie gebildet ist.

Vorteilhat ist, wenn das saugfähige, faserförmige Material aus einem oder mehreren von Materialien wie Rayon, Baumwolle, Zellstoff, Zellstoffwatte, Tissue-Schichtstoffe, Torfmull, Bambus oder chemisch verstärkte, modifizierte oder vernetzte Zellulosefasern, gebildet ist. Dieses faserförmige Material weist eine hohe Hygroskope auf und ermöglicht das Anhaften von hohen Flüssigkeitsmengen an den Außenseiten der Fasern und ist biologisch neutral..

Es ist aber auch möglich saugfähige, faserförmige Materialien zu verwenden, die durch eines oder mehrere der nachstehenden, synthetischen Materialien, wie Polyesterfasern, Polyolefinfasern, saugfähige Schaumstoffe, saugfähige Schwämme, saugfähige Polymere, kapillare Kanalfasern, synthetische Fasern, überwiegend offenzelligen Polyurethan-Weichschaum oder Fasern bzw. Fäden aus Rayon oder einem Strukturtyp der kristallen Modifikation von Zellulose II gebildet ist.

Bei einem Alltagstampon bzw. Tampon ist es weiters vorteilhaft, wenn der Kernbereich des Saugkörpers innerhalb eines Hüllkreises bzw. Hüllzylinders mit einem über seine Länge einen bevorzugt gleichen Durchmesser (21) ausgebildet ist, der bevorzugt kleiner 4 mm ausgebildet bzw. zwischen 4 mm und 1mm beträgt. Dadurch wird ein stabilerer Saugkörper geschaffen. Hierbei ist es vor allem dann auch von Vorteil, wenn im Kernbereich des Saugkörpers das saugfähige, faserförmige Material höher verdichtet ist als in den übrigen Bereichen des Saugköpers. Dadurch wird auch die Handhabung, dadurch dass der gesamte Körper stabiler wird, vereinfacht.

Von Vorteil ist es wenn der Alltagstampon bzw. Tampon am proximalen Ende rund oder abgerundet ausgebildet ist. Dadurch wird ein leichtes Einführen in eine Körperöffnung erzielt. Dies ist von Vorteil für Tamponerstanwender (z.B. junge Mädchen) und Frauen mit trockener Scheide.

Eine weitere vorteilhafte Weiterbildung sieht vor, dass der Saugkörper mit in Längsrichtung desselben verlaufenden Vertiefungen bzw. Rillen versehen ist und/oder dass die Vertiefungen bzw. Rillen in Richtung der Längsmittelachse des Saugkörpers wellenförmig, bevorzugt mit gleichbleibender Höhe der Amplitude verlaufen, wodurch eine Vergrösserung der Oberfläche des Tampons stattfindet. Beispielsweise ist dadurch eine vermehrte Aufnahme von Körperflüssigkeit möglich, da der Körper unterschiedliche Mengen an Körperflüssigkeit zwischen den aufeinanderfolgenden Menstruationsphasen, ausschütten kann. Ebenfalls sind diese Rillen bei der Verwendung von Einführhilfen für den Alltagstampon bzw. Tampon von Vorteil.

Eine noch höhere Oberfläche zur Feuchtigkeitsaufnahme kann dadurch erreicht werden, dass die Rillen bzw. Vertiefungen spiralförmig oder schraubenlinienförmig verlaufen.

Ein weiterer Vorteil wird dadurch erreicht, dass mehrere Rillen bzw. Vertiefungen in Umfangsrichtung über den Saugkörper verteilt angeordnet sind. Dadurch erfolgt ebenfalls eine Vergrößerung der Oberfläche des Tampons was zur vermehrten Aufnahme von Körperflüssigkeit führt, da der Körper unterschiedliche Mengen an Körperflüssigkeit zwischen den Menstruationsphasen ausschütten kann. Für Frauen mit vermehrtem Ausfluss ist eine größere Hygiene gegeben als bei einer Slipeinlage. Ebenfalls sind diese Rillen bei der Verwendung von Alltagstampon bzw. Tampons Einführhilfen von Vorteil.

Ein weiterer Vorteil bei der Verwendung des erfindungsgemäßen Alltagstampon bzw. Tampons ist die Kräftigung des Beckenbodens.

Zum besseren Verständnis der Erfindung wird diese anhand der in den nachfolgenden Figuren dargestellten Ausführungsbeispielen näher erläutert.

Es zeigen jeweils in stark schematisch vereinfachter Darstellung:
- Fig. 1: einen erfindungsgemäßen Tampon mit Vertiefungen wie in Seitenansicht;
- Fig. 2: den Tampon in Stirnansicht geschnitten gemäß den Linien II - II in Fig. 1;
- Fig. 3: eine andere Ausführungsvariante des Tampons mit einem sich konisch verjüngenden, distalen Ende und mit in Längsrichtung des Tamponkörpers wellenlinienverlaufenden Vertiefungen, wie zum Beispiel Rillen oder Nuten;
- Fig. 4: Stirnansichten des Tamponkörpers nach Fig. 3 in Stirnansicht geschnitten gemäß den Linien IV - IV;
- Fig. 5: Stirnansichten des Tamponkörpers nach Fig. 3 in Stirnansicht geschnitten gemäß den Linien V - V;
- Fig. 6: Stirnansichten des Tamponkörpers nach Fig. 3 in Stirnansicht geschnitten gemäß den Linien VI - VI;
- Fig. 7: eine Ausführungsvariante des erfindungsgemäßen Tampons mit parallel zur Längsachse des Tamponkörpers verlaufenden Vertiefungen und einer Vertiefung im Bereich des distalen Endes des Tampon;
- Fig. 8: eine Stirnansicht des Tamponkörpers nach Fig. 7 gemäß dem Pfeil VIII in Fig. 7;
- Fig. 9: eine Stirnansicht einer anderen Ausführungsform des Tamponkörpers geschnitten mit einer speziellen Ausgestaltung der äußeren Oberfläche des Tamponkörpers bildenden Rippen;
- Fig. 10: eine Stirnansicht einer anderen Ausführungsform des Tamponkörpers geschnitten mit einer speziellen Ausgestaltung der äußeren Oberfläche des Tamponkörpers bildenden Rippen;
- Fig. 11: einen Bandteil eines Bandes zur Herstellung eines Tamponkörpers für einen Tampon in schaubildlicher, vereinfachter, schematischer Darstellung;
- Fig. 12: die Anordnung von Bandteilen eines Bandes zur Herstellung eines pilzförmigen Tampons gemäß der Erfindung in schaubildlicher, schematischer Darstellung;
- Fig. 13: die Bandteile für den Saugkörper des erfindungsgemäßen Tampon in einer tulpenartig verformten Zwischenposition in vereinfachter, schematischer, schaubildlicher Darstellung;
- Fig. 14: den Saugkörper eines erfindungsgemäßen Tampons in fertigem Zustand in vereinfachter, schematischer, schaubildlicher Darstellung;
- Fig. 15: ein Band zur Herstellung eines erfindungsgemäßen Tampons mit den davon abgetrennten Bandteilen in schematischer, schaubildlicher Darstellung;
- Fig. 16: den vorgeformten Bandteil gemäß Fig. 15 mit den daran befestigten Auszugsmittel und den Werkzeugen zur Umformung des Bandteils in vereinfachter, schaubildlicher Darstellung;
- Fig. 17: einen fertig umgeformten, erfindungsgemäßen Tampon aus einem vorgefertigten Bandteil gemäß Fig. 16 in vereinfachter, schaubildlicher Darstellung.

Einführend sei festgehalten, dass in den unterschiedlich beschriebenen Ausführungsformen gleiche Teile mit gleichen Bezugszeichen bzw. gleichen Bauteilbezeichnungen versehen werden, wobei die in der gesamten Beschreibung enthaltenen Offenbarungen sinngemäß auf gleiche Teile mit gleichen Bezugszeichen bzw. gleichen Bauteilbezeichnungen übertragen werden können. Auch sind die in der Beschreibung gewählten Lageangaben, wie z.B. oben, unten, seitlich usw. auf die unmittelbar beschriebene sowie dargestellte Figur bezogen und sind bei einer Lageänderung sinngemäß auf die neue Lage zu übertragen. Weiteres können auch Einzelmerkmale oder Merkmalskombinationen aus den gezeigten und beschriebenen unterschiedlichen Ausführungsbeispielen für sich eigenständige, erfinderische oder erfindungsgemäße Lösungen darstellen.

Sämtliche Angaben zu Wertebereichen in gegenständlicher Beschreibung sind so zu verstehen, dass diese beliebige und alle Teilbereiche daraus mit umfassen, z.B. ist die Angabe 1 bis 10 so zu verstehen, dass sämtliche Teilbereiche, ausgehend von der unteren Grenze 1 und der oberen Grenze 10 mitumfasst sind, d.h. sämtliche Teilbereiche beginnen mit einer unteren Grenze von 1 oder größer und enden bei einer oberen Grenze von 10 oder weniger, z.B. 1 bis 1,7, oder 3,2 bis 8,1 oder 5,5 bis 10. Ein Wert innerhalb eines angegebenen Wertebereiches kann über eine Länge des Tampons gleichbleiben oder sich innerhalb dieser Grenzen verändern. Maximalwerde und Minimalwerte sind so zu verstehen, dass die Grenzwerte nach unten und oben durch die physikalischen und technischen Realisierungsmöglichkeiten definiert sind und z.B. bei Längen- oder Durchmesserangaben größer "null" und kleiner "unendlich" sind.

Die Ausführungsbeispiele zeigen mögliche Ausführungsvarianten des erfindungsgemässen Alltagstampon bzw. Tampons, wobei an dieser Stelle bemerkt sei, dass die Erfindung nicht auf die speziell dargestellten Ausführungsvarianten derselben eingeschränkt ist, sondern vielmehr auch diverse Kombinationen der einzelnen Ausführungsvarianten untereinander möglich sind und diese Variationsmöglichkeit aufgrund der Lehre zum technischen Handeln durch gegenständliche Erfindung im Können des auf diesem technischen Gebiet tätigen Fachmannes liegt. Es sind also auch sämtliche denkbaren Ausführungsvarianten, die durch Kombinationen einzelner Details der dargestellten und beschriebenen Ausführungsvariante möglich sind, vom Schutzumfang mit umfasst.

Der Ordnung halber sei abschließend darauf hingewiesen, dass zum besseren Verständnis des Aufbaus der Tampons diese bzw. deren Bestandteile teilweise unmaßstablich und/oder vergrößert und/oder verkleinert dargestellt wurden.

Die den eigenständigen erfinderischen Lösungen zugrundeliegende Aufgabe kann der Beschreibung entnommen werden.

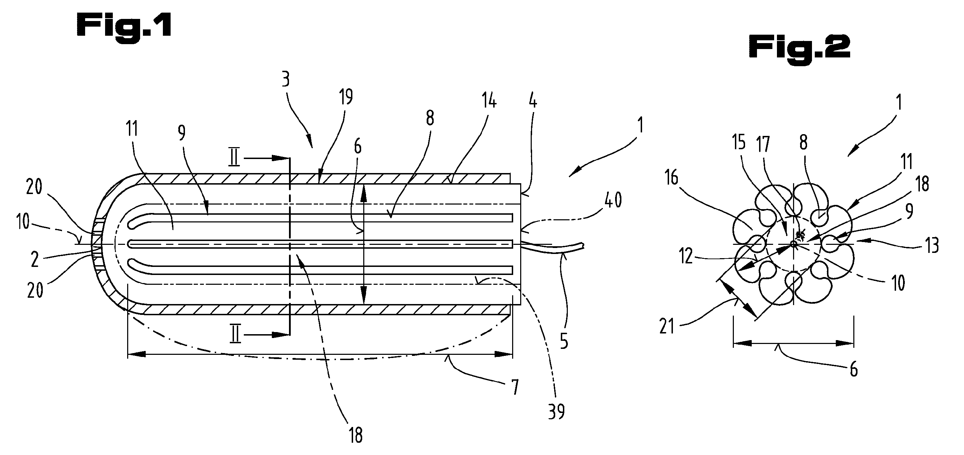

Die Figuren 1 und 2 zeigen einenTampon für die Zwischenmenstruationstage 1 mit einem kuppelförmigen proximalen Ende 2, einem länglichen, zylinderförmigen Mittelteil 3, einem distalen Ende 4 und einem dieses überragende Auszugsmittel 5, insbesondere einen Faden. Der zylinderförmige bzw. kegelstumpfförmige Mittelteil 3 erstreckt sich mit im Wesentlichen gleichbleibendem oder sich erweiterndem oder variierendem Durchmesser 6 bis zu seinem proximalen Ende 2. Zumindest über einen Teil seiner Länge 7 sind im Tampon bzw. im Mittelteil 3 z.B. durch Rillen 8 gebildete Vertiefungen 9 angeordnet. Ein äußerer Durchmesser 6 des Saugkörpers 16 bzw. eines den Saugkörper 16 umschließenden Hüllkörpers 37 kann einen Wert zwischen 2 mm und 14 mm, oder 6 bis 10 mm oder kleiner 8 mm betragen. Bei dem Hüllkörper handelt es sich um einen fiktiven Aussenmantel innerhalb dem sich der Außenumfang des Tampons 1 bzw. des Saugkörpers 16 zumindest teilweise anliegend oder tangierend befindet.

Der Maximalwert des Durchmessers kann einen Wert zwischen den angegebenen Wertegrenzen oder unterhalb der Wertegrenze betragen, wobei dieser Maximalwert über die gesamte Länge 7 des Tampon 1 oder zumindest über einen Teilbereich gleichbleibend sein kann oder gegebenenfalls auch über die Länge 7 des Tampons 1 bzw. einen Teil des selben innerhalb der angegebenen Wertegrenzen oder unterhalb eines Maximalwertes des Durchmessers variabel d.h. sich verändernd sein.

Wie aus Fig. 1 schematisch ersichtlich kann der Tampon 1 - siehe strichpunktierte Linie - im Mittelteil 3 eine umlaufende Vertiefung 38, zum Beispiel eine Rille oder eine Vertiefung mit einer beliebig anderen Raumform haben.

Zumindest ein Teil des Mittelteil 3 des Saugkörpers 16 oder ein diesen umhüllender Hüllkörper 37, oder der Tampon kann zylinderförmig ausgebildet sein. Es ist aber auch möglich, dass zumindest der Mittelteil 3 des Saugkörpers 16 oder des Tampons 1 bzw. ein diesen umhüllender Hüllkörper 37 kegel- bzw. kegelstumpfförmig ausgebildet ist.

Der Zylinder bzw. der Kegel bzw. der Kegelstumpf kann sich aber auch vom proximalen Ende bis zum distalen Ende durchgehend erstrecken.

Bei all den vorgenannten Raumformen des Saugkörpers 16 sind beliebige Querschnittsveränderungen über die zur Längsmittelachse 10 verlaufende Länge möglich, wie z.B. ein wellenförmiger Verlauf oder zumindest über einen Teil des Umfanges umlaufende oder sich in Längsrichtung erstreckende Vertiefungen die innerhalb des Hüllkörpers 37 angeordnet sind.

Der Begriff "distales Ende" 4, wie er hierin verwendet wird, bezieht sich auf jene Abschnitte der Anordnung und ihre Teile, die von dem Körper einer Benutzerin am weitesten entfernt sind, wenn der Tampon 1 in eine Körperöffnung, z.B. die Vagina, eingeführt wird. Der Begriff "proximales Ende" 2 bezieht sich auf jene Abschnitte der Anordnung und ihre Teile, die dem Körper einer Benutzerin am nächsten sind, wenn der Tampon eingeführt wird bzw. ist. Demzufolge geben die Begriffe "proximal" oder "distal", wie sie hierin verwendet werden, an, dass ein bestimmter Teil oder eine bestimmte Struktur der Anordnung oder ihre Teile dem proximalen Ende 2 bzw. dem distalen Ende 4 der Anordnung oder ihrer Teile näher ist. In ähnlicher Art und Weise beziehen sich die Begriffe "proximale Richtung" oder "distale Richtung" auf die Richtungen zum proximalen Ende 2 bzw. zum distalen Ende 4 des Tampons 1 hin.

Der Tampon 1 oder zumindest dessen Mittelteil 3 kann eine im wesentlichen zylindrische Form bzw. eine zylindrische Hüllfläche aufweisen. Ebenso ist es möglich, dass der Mittelteil sich vom Mittelteil 3 in distaler und oder proximaler Richtung verjüngt. Diese Verjüngung kann konisch oder mit einer beliebigen Raumform ausgebildet sein. Vor allem in proximaler Richtung kann der Mittelteil 3 sich auch erweitern.

Die Vertiefungen 9 an der Außenseite oder in der Oberfläche des Tampons 1 können durch einander unmittelbar benachbarte bzw. ineinander übergehende einen unterschiedlichen Abstand zur zentralen Längsmittelachse 10 des Tampons 1 angeordnete Bereiche gebildet sein, wobei die Bereiche 11 mit größerer Entfernung 12 zur Längsmittelachse 10 Rippen und die dazwischen liegenden Bereiche 13 Rillen aufweisen.

Die Dichte des, einen Saugkörper des Tampons 1 bildenden Materials, kann im Bereich 13 der Vertiefung 9 der Dichte im Bereich 11 der der Vertiefung 9 benachbarten Erhöhungen entsprechen. Es ist aber auch möglich, dass die Dichte im Bereich 13 der Vertiefungen 9 oder im Bereich 11 der Erhöhungen höher ist. Die Außenseite bzw. die Oberfläche 14 des Tampons 1 kann aber auch im Wesentlichen glatt ausgebildet sein.

Die Außenseite bzw. die Oberfläche 14 ist sowohl die Teilfläche, die von den Bereichen 13 mit radialer Vertiefung 9 z.B. den Rillen 8 gebildet werden, als auch die Teilflächen, die von den Bereichen 11 gebildet werden, die an diese Bereiche 13 angrenzen.

Eine Rille 8 stellt einen eindeutigen Bereich radialer Vertiefung 9 an der Außenseite des Tampons 1 dar der zumindest teilweise durch seine Länge, Breite, Tiefe und Ausrichtung definiert werden kann. Die Länge einer Rille 8 ist meistens größer als ihre Tiefe.

Jede der Vertiefungen 9 bzw. Rillen 8 kann über zumindest eine Teillänge der Länge 7 durchlaufen oder unterbrochen sein.

Der Tampon 1 bzw. dessen Mittelteil 3 kann beispielsweise zwischen 2 und 3 Vertiefungen 9 bzw. Rillen 8 aufweisen. Beispielhafte Tampons 1 können 2, 3, 4, 5, 6, 7, 8, 9,10, 11 oder 12 Rillen besitzen. Der Tampon 1 kann eine gerade oder eine ungerade Zahl von Vertiefungen 9 bzw. Rillen 8 besitzen. Wegen der Herstellungsanforderungen kann eine gerade Anzahl bevorzugt sein.

In einem weiteren Ausführungsbeispiel können die in Richtung der Längsmittelachse 10 des Alltagtampons 1 verlaufenden Vertiefungen 9 bzw. Rillen 8 in axialer Richtung spiralförmig oder schraubenförmig ausgebildet sein. Diese spiralförmigen oder schraubenförmigen Vertiefungen 9 bzw. Rillen 8 sind länger als jene Rillen die parallel zu der Längsmittelachse 10 des Tampons verlaufen. Diese Vertiefungen 9 bzw. Rillen 8 bedecken einen größeren Bereich der Oberfläche 14 bzw. der Außenseite 7. Durch die Anzahl der Vertiefungen 9 bzw. Rillen 8 deren Länge bzw. Tiefe kann die gesamte Oberfläche 14, die zur Aufnahme von Flüssigkeit herangezogen werden kann, an die entsprechende geforderte Leistungsfähigkeit angepasst werden. Vor allem wird dadurch auch die Weiterleitung der Flüssigkeiten in einen Zentralbereich 15 des Saugkörpers 16 des Tampons 1 an die unterschiedlichen Bedürfnisse einfach angepasst.

In diesem Zusammenhang ist es beispielsweise auch vorteilhaft, nach dem Einprägen der Vertiefungen 9, insbesondere der Rillen 8 in den Saugkörper 16 des Tampons 1, die dazwischen verbleibenden Bereiche 11 so zu verformen bzw. zu verdichten, dass, wie aus Fig. 2 besser ersichtlich, ein pilzförmigen Querschnitt erhalten wird. Dadurch wird erreicht, dass die Aufnahme von Flüssigkeiten bzw. das Anhaften derselben an den schematisch angedeuteten Fasern und Fäden 17 auch in einem Kernbereich 18 der zwischen den Vertiefungen 9 gebildet ist und auch in diesem Fall in den einen in etwa pilzförmigen Querschnitt aufweisenden Bereichen 11 erfolgen kann. Dies ist insbesondere bei den vorliegenden Tampon 1 von Vorteil, da dadurch bei einem geringeren Volumen des Saugkörpers 16 eine entsprechend rasche und hohe Feuchtigkeitsaufnahme vor allem in jenen Tagen zwischen den Menstruationsperioden erzielt werden kann.

Der maximale Aussendurchmesser 6 des Tampons 1 oder seines Mittelteils 3 kann in Längsrichtung im Wesentlichen gleichmäßig sein oder er kann variieren. Der Teil des Tampons 1 nahe an dem distalen Rückzugsende kann beispielsweise einen größeren maximalen Außendurchmesser 6 besitzen als der restliche Teil des Mittelteils 3. Durch dieses dicker werden am unteren Ende des Tampons 1 verringert sich die Gefahr, dass Körperflüssigkeit austritt, wenn der Tampon 1 in der Vagina platziert ist.

In einem Ausführungsbeispiel kann die Dichte des saugfähigen Materials über den Querschnitt des Tampons 1 im Wesentlichen gleich sein.

In einem anderen Ausführungsbeispiel kann der Zentralbereich 15 des Tampons 1 bzw. des Saugkörpers 16 aus stark komprimiertem, saugfähigem, faserförmigem Material bestehen.

Im Übrigen ist es auch möglich, einen Zentralbereich 15 sehr hoch zu verdichten und dagegen die zwischen den Vertiefungen 9 bzw. Rillen 8 liegenden Bereiche 13 geringer zu verdichten, sodass diese ein höheres selbsttätiges, elastisches Rückstellvermögen haben. Diese geringere Dichte und die höhere Elastizität in diesen zwischen den Vertiefungen 9 bzw. Rillen 8 liegenden Bereichen 11 kann die Druckbelastung auf jene Körperteile an denen der Tampon 1 anliegt, reduziert werden und darüber hinaus ein höherer Tragekomfort mit einem angenehmeren Trageempfinden für die Benutzerin durch die den Bewegungen der an dem Saugkörper 16 anliegenden Körperteilen besser nachfolgende Ausgestaltung der zwischen den Vertiefungen 9 bzw. Rillen 8 gebildeten Rippen bzw. Bereiche 11 erreicht werden.

Der vorliegende Tampon 1 besitzt ein verengtes proximales Einschubende 2, d.h. ein Einschubende mit einem kleineren Durchmesser im Querschnitt als der Durchmesser 6 des Mittelteils 3 des Tampons 1. Das Einschubende kann beispielsweise konisch oder abgerundet sein und es kann vorzugsweise eine abgerundete Kuppelform besitzen. Durch die Ausbildung des proximalen Endes 2 kann der Tampon 1 außerdem tiefer in die Vagina eingeführt werden, d.h. näher an den Gebärmutterhals, was das Befeuchten des Tampons 1 vorteilhafterweise fördert, da sich die aufzufangende Flüssigkeit tief in der Vagina befindet. Tampons 1 mit einem verengten Einschubende , insbesondere einem abgerundeten oder runden, kuppelförmigen proximalen Ende 2, werden von den Verbraucherinnen deshalb im Allgemeinen bevorzugt.

Saugfähiges Fasermaterial für den Tampon 1 bzw. dessen Saugkörper 16 kann aus jedem beliebigen, saugfähigen Material 36 bestehen, das eine gute Saugfähigkeit und Elastizitätsmodul besitzt, welche Flüssigkeit aufnehmen und/oder zurückhalten können. Die saugfähige Struktur lässt sich aus einer Vielzahl von Größen und Formen und aus einer Vielzahl von flüssigkeitsabsorbierenden Materialien 36 herstellen.

Selbstverständlich ist es wünschenswert, saugfähige Materialien zu verwenden, die einen Mindestgehalt an fremdlöslichen Materialien enthalten, da das Produkt für einen bestimmten Zeitraum im Körper verbleibt. Zurückgehaltene lösliche Fremdmaterialien könnten ein Sicherheitsrisiko darstellen, wenn sie toxisch oder reizend sind.

Eine Liste brauchbarer Materialien enthält zellulosehaltige Materialien wie beispielsweise Rayon, Baumwolle, Zellstoff, Zellstoffwatte, Tissue-Schichstoffe, Torfmull, Bambus und chemisch verstärkte, modifizierte oder vernetzte Zellulosefasern; synthetische Materialien wie beispielsweise Polyesterfasern, Polyolefinfasern, saugfähige Schaumstoffe, beispielsweise ein elastisch federnder Polyurethanschaumstoff, saugfähige Schwämme äußerst, saugfähige Polymere, saugfähige, gelbildende Materialien, bearbeitete Fasern wie beispielsweise kapillare Kanalfasern und Fasern mit mehreren Gliedern; synthetische Fasern oder ein äquivalentes Material oder Kombinationen von Materialien oder Mischungen daraus.

Bei Verwendung derartiger Kunststoffschäume ist es auch möglich, beispielsweise durch Temperatureinstellung bei der Herstellung der Saugkörper partiell die Außenhaut zu verschließen, das heißt im Außenbereich aufgrund partieller Temperaturbeeinflussung eine geschlossene Haut zu erzielen. Dadurch kann auch ein Austritt von in der Schaumstruktur aufgenommener Flüssigkeit zusätzlich erschwert oder verhindert werden. Eine derartige Ausbildung kann vor allem im distalen Endbereich 4 des Tampons 1 von Vorteil sein.

Die Herstellung des Tampons 1 kann im Rahmen der Erfindung aus unterschiedlichsten Materialien erfolgen, die unter den Bedingungen im Bereich der Scheide bzw. Gebärmutter, das heißt bei Körpertemperaturen einen pH-Wert von ca. 4 über längere Zeit ihren Zustand aufrecht erhalten und keine Giftstoffe oder schleimhautschädlichen Lösungen oder dergleichen abgeben. Vor allem Polyurethanweichschäume mit sehr niedrigem Raumgewicht und einer überwiegend offenzelligen Struktur können mit Vorteilen eingesetzt werden. Durch die Verwendung von absorptionsfähigerem Material wird auch die Gefahr des Austretens von Flüssigkeit weiter reduziert.

Der Vorteil für derartige, schaumförmige Strukturen liegt auch darin, dass die offenen Zellen im Auslieferungszustand oder vor dem Einführen in die Scheide mit Medikamenten bzw. Gleitmittel gefüllt werden können, die im eingesetzten Zustand an die Schleimhäute der Scheide bzw. des Uterus abgegeben werden können.

In einer Ausführungsart kann die Oberfläche des Tampons 1 mindestens teilweise mit einer zumindest teilweise flüssigkeitsdurchlässigen Umhüllung 19 versehen werden. Die Umhüllung 19 liefert eine glattere Oberfläche. Dadurch verringert sich das Risiko, dass sich während der Verwendung Fasern von der Oberfläche 14 des Tampons 1 lösen. Die Wahrscheinlichkeit dass Tamponmaterial verloren geht, verringert sich ebenfalls. Die Umhüllung 19 verringert das Absorptionsvermögen und die Saugfähigkeit oder die Ausdehnungsfähigkeit des Tampons 1 meistens nicht. Die Verwendung von Umhüllungen bei Tampons ist in dem Fachgebiet wohl bekannt, ebenso wie die geeigneten Materialien für diese Umhüllungen. Die Umhüllung kann am proximalen Einschubende 2 bzw. der Stirnseite des Tampons 1 wegfallen, damit die Körperflüssigkeit dort besser aufgenommen werden kann, oder zum Beispiel zumindest in diesem Bereich mit Durchbrüchen oder Öffnungen 20 versehen sein.

Von Vorteil ist es weiteres, wenn die verwendeten Materialien biologisch abbaubar sind und beispielsweise aus PLA oder sonstigen, biologisch abbaubaren Kunststoffen oder Kunststoffgemischen bzw. Fäden oder Fasern bestehen oder recyclierten Kunststoffe, wie R-PP, R-PET oder dergleichen gegebenenfalls in unterschiedlichen Mischungen oder als mehrlagige Teile aus diesen Materialien 36 gebildet sein.

Bei dem Tampon 1 ist es in Anbetracht der oftmaligen Anwendung zwischen den Menstruationsphasen bzw. -perioden, gegebenenfalls sogar täglichen Benutzung, von Vorteil, wenn die Schleimhäute im Bereich der Vagina, sowie deren zirkulär verlaufende Muskeln, möglichst gering belastet sind. Vor allem sind daher die Maßnahmen von Vorteil, bei welchen der Druck auf die Schleimhäute, auch in entspanntem Zustand der Vagina, gering ist und vor allem Erosionen der Schleimhaut und/oder eine Schädigung und/oder nachteilige Veränderung des pH-Wertes im Bereich der Schleimhäute verringert bzw. überhaupt vermieden wird.

Dazu hat es sich als vorteilhaft erwiesen, die Länge 7 des Alltampons 1 und/oder dessen Durchmessers 6 und/oder dessen Volumens und/oder dessen Gewichts in ein bestimmtes Verhältnis zu setzen.

So ist es beispielsweise vorteilhaft, wenn ein Durchmesser 6 des Tampons 1 einen Wert zwischen 6 und 14 mm, bevorzugt kleiner als 10 mm, beispielsweise kleiner als 8 mm aufweist.

Durch die Wahl des Wertes des Durchmessers 6 des Tampons 1 können diese in einfacher Weise an unterschiedliche anatomische Voraussetzungen bei unterschiedlichen Personen angepasst werden. Darüber hinaus ist es auch möglich, durch die Größe des Durchmessers das Volumen der saugfähigen Materialien und dadurch die Aufnahme von Flüssigkeit so zu bemessen, dass je nach den unterschiedlichen Bedingungen bei unterschiedlichen Personen Tampons 1 mit unterschiedlicher Feuchtigkeitsaufnahme hergestellt und angepasst an die Bedürfnisse unterschiedlicher Personen eingesetzt werden können. Hierbei ist es dann, wenn ein höheres Aufnahmevermögen von Flüssigkeit erforderlich ist, möglich, den Saugkörper 16 über sein gesamtes Volumen aus einem saugfähigen Material herzustellen. Sollen Tampons 1 mit einer geringeren Saugfähigkeit hergestellt werden, ist es auch möglich, einen Zentralteil 15 bzw. einen Kernbereich 18 entweder aus faserförmigem Material oder anderen, beispielsweise feuchtigkeitsabweisenden, Materialien herzustellen. Werden in dem Kernbereich 18 bzw. Zentralbereich 15 faserförmige Materialien angeordnet, ist es auch möglich, dass diese bei gleichem Volumen zur Aufnahme einer geringeren Flüssigkeitsmenge ausgebildet sind als das Material 36 diesen Zentral- bzw. Kernbereichs 15, 18 umschließenden Materials des Saugkörpers 16.

Durch die Verwendung eines derartigen Zentral- bzw. Kernbereiches 15, 18 ist es auch möglich, die Menge des saugfähigen Materials 36 des Saugkörpers 16 zu verändern und anzupassen, sodass dann, wenn ein größerer Außendurchmesser des Tampon 1 aus anatomischen Gründen erforderlich ist, jedoch dieser eine geringere Aufnahmefähigkeit von Flüssigkeiten aufweisen soll, in dem Zentral- bzw. Kernbereich 15, 18 Materialien vorgesehen werden können, die eine äußerst geringe oder überhaupt keine Feuchtigkeitsaufnahme aufweisen.

Bei derartigen Ausführungsformen ist es auch zu empfehlen, dass das Auszugsmittel 5 entweder mit dem Saugkörper 16 und/oder mit dem Zentral- bzw. Kernbereichs 15, 18 des Tampons verbunden ist. Dadurch kann auch ein sicheres Ausziehen des Tampons erreicht werden, wenn die äußere Hülle des Saugkörpers 16, die den Zentral- bzw. Kernbereich 15,18 umgibt, aus gering verdichteten Fasern bzw. aus einer dünnen Lage bzw. einer Lage mit geringer Reißfestigkeit besteht.

Die Länge 7 eines Tampons 1 ist kleiner 40 mm und weist bevorzugt einen Mindestdurchmesser von 5 mm auf. Ein günstiger Durchmesser für einen derartigen Tampon 1 für die Verwendung an den Tagen zwischen den Menstruationsperioden ist eine Länge 7 zwischen 30 und 38 mm, aber für bestimmte Anwendungsfälle ist es aber auch möglich, dass die Länge 7 zwischen 30 mm und 10 mm beträgt. Dies bedeutet, dass die Länge 7 jeden Wert zwischen 10 und 30 mm annehmen kann. Das gleiche gilt auch für die zuvor angegebenen Längenwerte.

Bei den zuvor genannten Abmessungen dieses Tampons 1 ist es vorteilhaft, wenn das Flächengewicht des für die Herstellung des Tampons 1 verwendeten Bandes bezogen auf eine Breite, die bevorzugt in etwa der Länge des Tampons 1 entspricht, zwischen einem Mindestwert von 1 g/lfm und 30 g/lfm gewählt wird. Dadurch kann ein Gewicht des Tampons 1 ohne dem Auszugsmittel 5 von unter 2 g, vorzugsweise unter 1,7 g, beispielsweise aber auch zwischen 1,5 und 0,5 g erzielt werden.

Bei den zuvor genannten, digitalen Tampons 1 die üblicherweise einen verdichteten Kernbereich 18 des Saugkörpers 16 aufweisen, empfiehlt sich ein Durchmesser 21 für den höher verdichteten Kernbereich 15 des Saugkörpers 16 von Werten unter 6 mm, bevorzugt unter 4 mm bis zu einem unteren Wert von 0,5 mm.

Dadurch steht in den über den Umfang des Zentral- bzw. Kernbereichs 15, 18 vorragenden Bereichen des Saugkörpers 16 ein weniger verdichtetes Fasermaterial zur Verfügung, welches eine höhere Saugwirkung und bessere Kapillarwirkung aufweist und an dem die Flüssigkeitstropfen besser anhaften können.

Des Weiteren kann es sich auch als vorteilhaft erweisen, dass der Tampon 1, insbesondere dessen Saugkörper 16 eine höhere Elastizität bei Verformungen um senkrecht zur Längsmittelachse 10 verlaufende Achsen ermöglicht und kann sich daher besser den Gegebenheiten im Inneren des Körpers der Benutzerin anpassen.

Dazu kommt, das in vorteilhafter weise durch die Anpassung der Elastizität des Saugkörpers 16 in radialer Richtung zu seiner Längsachse 10 eine Anpassung an unterschiedliche Empfindlichkeiten von benutzenden Personen insofern möglich ist, dass die dadurch bewirkte Druckwirkung auf die Körperteile entsprechend variabel ausgestaltet werden kann. Dadurch können Belastungen, Irritationen der Schleimhäute an die Gegebenheiten bei verschiedenen Personen einfach angepasst werden und können unterschiedliche Tampons 1 für unterschiedliche Bedürfnisse hergestellt werden.

Dazu kommt, dass dadurch auch das Gewicht des Saugkörpers 16 ohne dem Auszugmittel 5 entsprechend reduziert werden kann, und damit die Zugbelastung auf die am Saugkörper anliegenden Körperteile der Benutzerin entsprechend reduziert werden kann, und zwar sowohl im trockenen Zustand als auch nach Aufnahme von Flüssigkeit bzw. Feuchtigkeit in feuchtem Zustand. Damit können aber auch die nach unten gerichteten Kräfte auf den Sphinctermuskel der Blase im Bereich des Blasenausganges reduziert werden.

Vor allem bei derartigen Tampons, bei welchen die aufzunehmende Flüssigkeit in geringeren Mengen pro Zeiteinheit erfolgt, ist eine derartige geringer verdichtete Struktur in einem größeren Querschnittsbereich des Saugkörpers 16 von Vorteil.

Je nach dem verwendeten Material für den Saugkörper, beispielsweise durch Verwendung von Rayonfasern oder Fasern bzw. Fäden aus Zellulose, beispielsweise durch einen Strukturtyp der Kristallmodifikation von Zellulose II, kann diese Absorptionsmenge des Saugkörpers 16 noch zusätzlich erhöht werden und dadurch das Volumen kleiner gehalten werden, wodurch in vorteilhafter Weise die Aufnahmekapazität des Saugkörpers 16 kleiner 4 ml erzielt werden kann.

Die Aufnahmekapazität des Saugkörpers 16 auf sehr geringe Werte kann aber wie bereits zuvor erwähnt auch dadurch erfolgen, dass im Zentral- bzw. Kernbereich 15, 18 Bereiche mit äußerst geringer Feuchtigkeitsaufnahme oder überhaupt flüssigkeitsabweisende Materialien angeordnet werden. Dadurch ist auch in einfacher Weise eine Variation der Absorptionsmenge der Flüssigkeit durch den jeweiligen Saugkörper 16 erzielbar. Als vorteilhaft hat sich eine Absorptionsmenge von Flüssigkeit von 4 ml bzw. weniger als 4 ml herausgestellt. Nachdem aber bei unterschiedlichen Personen die an den unterschiedlichen Tagen abgegebenen Flüssigkeiten auch variieren können, ist es auch vorteilhaft, derartige Tampons 1 herzustellen, die eine Absorptionsmenge von Flüssigkeit von 3 bis 3,5 ml oder von lediglich 2 ml aufweisen.

Bei den Angaben dieser Werte wird davon ausgegangen, dass die Angaben der Flüssigkeitsmenge in ml mit den Gewichtsangaben in g im Wesentlichen übereinstimmen.

Der Tampon 1 besitzt im Allgemeinen ein Auszugsmittel 5 wie einen Rückziehfaden der über das proximale Entnahmeende des Tampons 1 vorkragt und im Saugkörper des Tampons 1 festgelegt ist, um ein Herausziehen des Tampons 1 nach dem Gebrauch zu erleichtern. Das Auszugsmittel 5 kann vorzugsweise elastisch und wasserabweisend sein und muss genug Spannung aufweisen, damit er beim Herausnehmen des Tampons 1 nicht reißt. Er kann aus beliebigen Materialien hergestellt sein, die im Stand der Technik für Rückziehfäden verwendet werden. Es kann sich dabei um eine einzelne Schnur, ein Band oder eine Vielzahl von Fäden handeln. Ein wasserabweisender Baumwollfaden, ein wasserabweisender Polyesterfaden oder eine Mischung daraus sind Materialien, die sich als Auszugsmittel 5 bzw. Rückziehfaden bewährt haben. Mit Polyester kann das Auszugsmittel stärker werden. Das Auszugsmittel 5 kann auf beliebige Art und Weise, die den Fachleuten auf dem Gebiet bekannt ist, an dem Tampon 1 befestigt werden. So ist es, wie bereits einleitend angegeben, möglich, dass das Auszugsmittel bzw. der Rückziehfaden an dem Bandteil aus dem der Saugkörper 16 des Tampon 1 hergestellt ist, angenäht ist oder die beiden Streifenteile die den Saugkörper 16 bilden, von dem Auszugmittel 5 zumindest an zwei voneinander distanzierten Stellen durchsetzt sind und innerhalb einer Schlaufe dieses Auszugsmittel 5 angeordnet sind. Das Auszugmittel 5 kann aber auch eine Schlaufe bilden, durch die der Bandteil des Saugkörpers 16 hergestellt wird, durchsetzt ist. Auch das Einweben, Anschweißen, Ankleben des Auszugsmittels an dem Saugkörper 16 bzw. den diesen herstellenden Bandteilen ist ebenso möglich.

Eine weitere Möglichkeit ist im Inneren eines Tampon 1 bzw. Saugkörpers 16 den Wirkstoff zu platzieren, und sobald dieser feucht wird, wird der Wirkstoff freigesetzt. Dies würde einer Retardfreisetzung entsprechen.

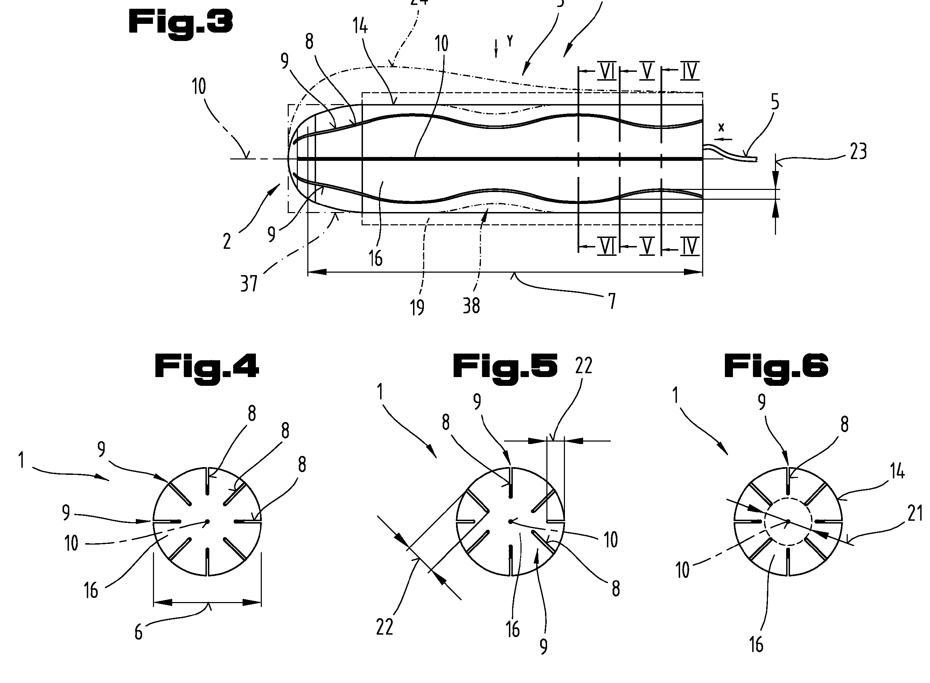

Eine weitere Ausführungsform eines Tampons 1 ist in den Fig. 3 bis 6 gezeigt. Dieser weist einen länglichen Saugkörper 16 mit einem sich über einen Teil seiner Länge 7 konisch verjüngenden und kuppelartig ausgebildeten, proximalen Ende 2 auf. Über die Länge 7 des Tampons 1 sind an der Außenseite bzw. in der Oberfläche 14 Vertiefungen 9, beispielsweise Rillen 8, angeordnet. Diese Vertiefungen 9, insbesondere Rillen 8, können einen zur Längsmittelachse 10 wellenförmigen Verlauf aufweisen und zwar sowohl in Umfangsrichtung des Saugkörpers 16 als auch in der Tiefe 22, wie dies schematisch anhand der Abbildungen in den Stirnansichten in verschiedenen in Längsrichtung der Längsmittelachse 10 distanzierten Bereichen in den Fig. 4 bis 6 gezeigt ist. Eine Höhe 23 der Amplitude dieses wellenförmigen Verlaufes kann über die Länge 7 des Saugkörpers 16 gleichmäßig verlaufen bzw. kann jedoch über die Länge 7 unterschiedliche Werte aufweisen.

Gleiches gilt für den wellenförmigen Verlauf der Tiefe 22, wobei die Höhe der Amplitude für den wellenförmigen Verlauf der Vertiefungen 9 bzw. Rillen 8 ebenfalls über die Länge 7 des Saugkörpers 16 oder einen Teil desselben gleichförmig verlaufen kann oder in unterschiedlichen Bereichen, beispielsweise im Mittelteil 3 oder im Bereich der Enden 2, 4 unterschiedlich groß sein kann.

Viele Tampons, die als Digitaltampon eingesetzt werden, sollen also die manuell durch die Benutzerin selbst in die Vagina eingeführt werden, empfiehlt es sich hierbei, wenn der Zentral- bzw. Kernbereich 15,18, der einen Durchmesser 21 aufweisen kann, eine stabförmige Stützfunktion für den Tampon 1 bewirkt, indem er stärker verdichtet ist.

Ist nun beispielsweise der Tampon 1 bei dieser Ausführungsform nicht mit einer Umhüllung 19, beispielsweise aus einem Nonwoven überzogen, so wird bzw. erstreckt sich diese Umhüllung 19 nur über einen Teil der Länge 7 des Tampons 1, wie dies mit strichlierten Linien schematisch in Fig. 3 angedeutet ist, so quillt der Saugkörper 16 bei Benetzung vom proximalen Ende 2 her in radialer Richtung auf und nimmt eine Raumform ein, wie sie rein schematisch und stark übertrieben durch die strichpunktierte Linie 24 angedeutet ist. Ist die Umhüllung 19 über den dargestellten Teilbereich der Länge 7 des Tampons 1 beispielsweise nur über die Länge des Mittelteils 3 vorgesehen, so wird dadurch das Aufquellen in radialer Richtung im Mittelbereich stärker reduziert, als wenn diese Umhüllung 19 nicht vorgesehen ist.

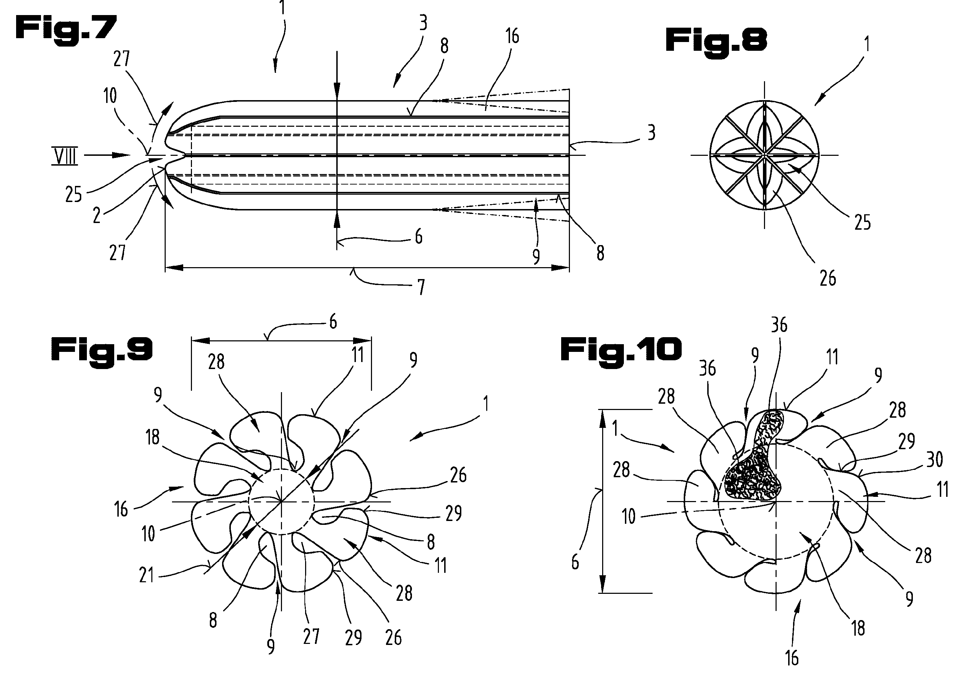

Eine andere Ausführungsvariante eines erfindungsgemäßen Tampons 1 ist aus den Fig. 7 und 8 ersichtlich. Dieser Tampon 1 bzw. dessen Saugkörper 16 weist in Richtung der Längsmittelachse 10 verlaufende Vertiefungen 9 bzw. Längsrillen 8 auf. Der Mittelteil 3 des Saugkörpers 16 verläuft anschließend eine an den konisch in Richtung des proximalen Endes 2 verjüngenden Endbereich einen zylindrischen Durchmesser auf, ist es jedoch ebenso möglich, dass ein Durchmesser 6 im Bereich des distalen Endes 4, wie mit strichpunktierten Linien angedeutet, kleiner oder größer sein kann als im Mittelteil 3.

Auch der Verlauf der Vertiefungen 9 muss nicht geradlinig und parallel zur Längsmittelachse 10 verlaufen, sondern kann, wie beispielsweise in Fig. 3 dargestellt, einen wellenförmigen Verlauf sowohl in Umfangsrichtung des Saugkörpers 16 als auch in Richtung der Tiefe 22 aufweisen. Unter anderem ist es auch möglich, dass die Vertiefungen zum Beispiel in Art eines Gewindeganges spiralförmig verlaufen und sich zumindest über einen Teil der Länge des Mittelteils 3 erstrecken.

Zudem kommt, dass im Bereich des distalen Endes 2 der Saugkörper 16 mit einer Ausnehmung 25 versehen sein kann.

Die vorragenden blattförmigen Endbereiche 26 des Saugkörpers können dabei auch so gestaltet werden, dass es sich beispielsweise unter der Einwirkung von Feuchtigkeit nach außen, also in Richtung der Pfeile 27, ähnlich den Blütenblättern einer Tulpe öffnen, um somit eine zusätzliche Auffangzone bzw. einen Auffangraum bilden, in dem sich die aus dem Körperinneren kommenden Flüssigkeit sammeln kann, um dann in den einzelnen Vertiefungen 9 bzw. Rillen 8 über die Länge 7 des Saugkörpers 16 verteilt aufgesaugt zu werden.

Die Fig. 9 und 10 zeigen des Weiteren unterschiedliche Ausgestaltungen der Querschnittsform der neben den Vertiefungen 9 verbleibenden, rippenartig vorstehenden Bereiche 11. So weisen die Rippen 28 einen L- bzw. schuhförmigen Querschnitt in der in Fig. 9 gezeigten Draufsicht auf. Dagegen liegen die stirnseitigen Enden 29 der Bereiche 11 an den rückseitigen Endbereiche 30 nahezu an und es wird zwischen diesen ein Hohlraum 31 gebildet bzw. von diesen umschlossen, der sich zumindest über einen Teil der Länge 7 des Saugkörpers 16 erstreckt. Dadurch wird eine große Oberfläche 14 des Saugkörpers 16 zur Aufnahme von Feuchtigkeit bzw. Flüssigkeit geschaffen und auch sichergestellt, dass die vom distalen Ende 2 weiter entfernten Bereiche des Saugkörpers 16 entsprechende Feuchtigkeit oder Flüssigkeit zugeführt und durch diese aufgenommen werden kann, sodass die Absorptionskapazität des Tampon 1 optimal ausgenutzt werden kann.

Bei der Ausführungsvariante gemäß Fig. 10 ist vorgesehen, dass die während der Herstellung der Vertiefungen 9 etwa radial nach außen vorragenden Rippen 28 in radialer Richtung umgebogen und so verformt werden, dass sie eine umlaufende, äußere Hüllfläche des Tampon 1 bilden. Die Endbereiche 29 der Rippen 28 liegen dabei direkt an den Endbereichen 30 der benachbarten Rippe 28 an. Durch diese radiale Umformung bildet die Vertiefung 8 einen längeren, kapillarartigen Kanal der eine höhere Saugwirkung in das Innere des Saugkörpers 16 ausüben kann.

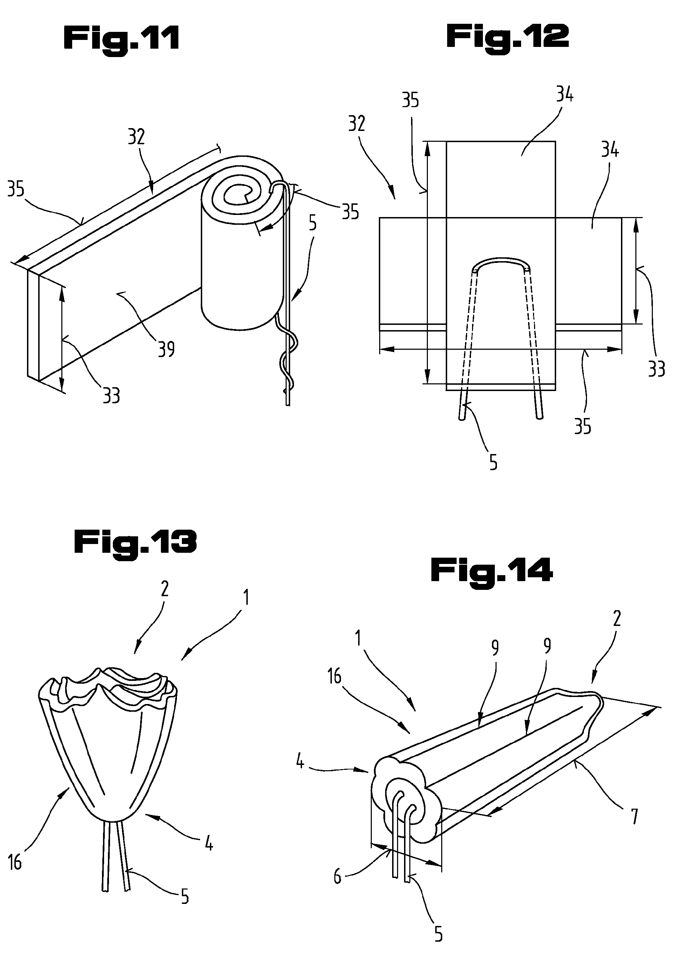

In Fig. 11 ist der für die Herstellung des Saugkörpers 16 während der Produktion des Tampons 1 vorgefertigte Wickel in halbfertigen Zustand gezeigt. Derartige Wickel werden vor allem für die sogenannten Digitaltampons eingesetzt. Diese bestehen aus einem Band 32, welches eine Breite 33 von 20 bis 50 mm, bevorzugt kleiner 38 mm, z.B. 15 bis 30 mm aufweist. Von diesem Band werden wie dargestellt, Bandteile 34 mit einer abgewickelten, gestreckten Länge 35, die in Abhängigkeit von einem gewünschten Volumen des Saugkörpers 16 bzw. dem geplanten Durchmesser 6 und/oder dem Laufmetergewicht des Bandes 32 des Tampons 1 ermittelt wird, ab gelängt. Bei dem Tampon, kann die Länge ca. 250 mm oder kürzer sein, z.B., aber auch zwischen 80 und 150 mm, bevorzugt zwischen 100 und 120 mm betragen.

Das Laufmetergewicht eines derartigen Bandes mit einer der Breite 33 des Zuschnitts des Bandteils 34 zur Herstellung eines Tampons entsprechenden Breite beträgt lediglich zwischen 1 und 30 g. Diese Tampons in Form von Digitaltampons werden derart hergestellt, dass der längliche Bandteil 34 um eine quer zur Längsrichtung des Bandes 32 verlaufende Achse zu einem zylindrischen Körper zusammengerollt wird. Der zylindrische Körper wird dann in radialer Richtung verdichtet und einer Formgebung unterzogen, sodass gegebenenfalls eines, nämlich das distale Ende 2, mit einem sich konusförmig verjüngenden, abgerundeten Ende oder einem kugel- oder kugelkalottenförmigen Endbereich, wie beispielsweise in den Fig. 1 bis 10 dargestellt, ausgebildet ist. Das gegenüberliegende Ende, aus welchem das um das Band vor dem Zusammenrollen herumgewickelte und über die Breitseite des Bandes vorragende Auszugmittel 5, zum Beispiel der Rückziehfaden, vorragt, wird mit einem flächigen Abschluss oder einer konkav gewölbten Stirnfläche ausgebildet.

Üblicherweise werden bei der Herstellung von Digitaltampons, wie in den Fig. 1 bis 10 beschrieben, einzelne Längsbereiche des zylinderförmigen Rohkörpers stärker verdichtet, sodass ein versteifter Zentral- bzw. Kernbereich 15, 18 entsteht und die darüber vorragenden zwischen diesen Pressnuten verbleibenden Tamponbereiche eine geringere Verdichtung und damit eine höhere Elastizität aufweisen.

Für diese Herstellung dieser Tampons können, wie für die bereits vorher erwähnten, unterschiedlichen Typen der Tampons, alle beliebigen aus dem Stand der Technik bekannten Materialien 36, die wie in Fig. 10 schematisch angedeutet durch mehr oder weniger stark verdichtete Fasern oder Fäden bzw. Zellen gebildet sein können, verwendet werden.

Anstelle der stärkeren Verdichtung einzelner Teile des zylinderförmigen Rohkörpers des Saugkörpers 16 kann der Zentral- bzw. Kernbereich 15,18, wie beispielsweise in Fig. 1 mit zweistrich punktierten Linien angedeutet, auch durch einen eingelegten Formkörper gebildet sein. Dieser eingelegter Formkörper kann aus einem feuchtigkeitsabweisenden Material sein, aber es ist ebenso möglich, dass dieser auch aus einem faserförmigen Material 36 gebildet wird, welches durch entsprechende Behandlung, z.B. Verdichtung oder durch Verwendung der entsprechenden Materialien eine geringere Flüssigkeitsmenge pro Volumeneinheit aufweisen kann als die gleiche Volumeneinheit aus dem Material 36 des Saugkörpers 16. Ebenso ist es möglich, dass der Rohkörper aus einem feuchtigkeitsabweisenden Material, beispielsweise aus Kunststoffteil, ausgebildet ist.

Dieser Rohkörper bzw. dieser dadurch gebildeter Zentral- bzw. Kernbereich 15,18 ist dann von dem Saugkörper 26 zumindest im Bereich seines proximalen Endes 2 als auch im Bereich des Mittelteils 3 vom Saugkörper umschlossen, wobei der Saugkörper vorzugsweise durch eine zylinderförmige Höhle gebildet sein kann, die in dem das proximale Ende bildenden Bereich verschlossen und auf der dem distalen Ende 4 des Tampons 1 gegenüberliegendem Ende offen oder mit einer Öffnung versehen ist, sodass ein Rohkörper, der den Zentral- bzw. Kernbereich 15,18 bildet, in diese einem Fingerling entsprechende Hülle eingebracht werden kann.

Durch das Volumen des eine geringe oder nahezu keine Feuchtigkeit aufnehmenden Zentral- bzw. Kernbereich 15, 18 kann die Menge an Flüssigkeit, die durch diesen Saugkörper 16 aufgenommen werden kann, unabhängig vom Gesamtdurchmesser des Tampons 1 an unterschiedliche Anwendungsfälle einfach angepasst werden. Damit wird eine hohe Flexibilität sowohl hinsichtlich der Menge an Flüssigkeit, die von dem Tampon 1 aufgenommen werden kann einerseits, und zwar unabhängig von den aus anatomischen Gründen bei unterschiedlichen Personen benötigten unterschiedlichen Durchmessern der Zentral- bzw. Kernbereiche 15, 18 - die unterschiedlichsten Raumformen aufweisen können -,erreicht. Bei der Herstellung des Saugkörpers 16 mit einer radialen elastischen Vorspannung kann sich dieser beim Überziehen des diesen Zentral- bzw. Kernbereich 15, 18 bildenden Rohkörpers an dessen Außenform anpassen und können somit auch unterschiedlichste Außenformen von erfindungsgemäßen Tampons 1 hergestellt werden.

Der Saugkörper 16 weist somit einen Hohlraum 39 auf, der über eine Öffnung 40 in dem, dem distalen Endbereich 4 zugewandten Ende bzw. dem das proximale Ende 2 bildenden Endbereich gegenüberliegend, im Saugkörper 16 angeordnet ist, zugänglich ist.

Zudem ist es möglich, dass die einzelnen Tampons 1 mit sogenannten Nonwoven überzogen sind bzw. mit sonstigen, netzartigen oder Durchbrüchen aufweisenden Überzügen versehen sind, die einen Faserverlust und insbesondere eine erhöhte Reibung zwischen den Schleimhäuten der Vagina und der äußeren Oberfläche des Tampons verringern bzw. einen Faserverlust vermeiden sollen.

In den Fig. 12 bis 14 ist eine unterschiedliche Art eines Tampons 1, basierend auf einer zu der zuvor beschriebenen Herstellungsart unterschiedlichen Herstellungsart dargestellt und beschrieben. Es handelt sich bei dieser Ausführungsform des Tampons 1 um einen sogenannten Pilzkopftampons, bei welchen zwei etwa rechteckförmige Bandteile 34 mit einer Länge 35 zwischen 40 bis 80 mm und einer Breite 33 von 10 bis 30 mm mit ihren Längsmittelachsen jeweils 90° versetzt übereinander gelegt werden. Im Bereich der einander übereinanderliegenden Schnittpunkte der strichpunktiert dargestellten Diagonalen der beiden Bandteile 35 benachbart, wird eine Schlinge eines Auszugmittels 5 durch die beiden Bandteile 35 hindurchgezogen. Die freien Enden der beiden Bandteile 35 werden dann tulpenblütenartig zusammengefaltet - siehe Fig. 13 - sodass ein im Wesentlichen zylindrischer Körper entsteht, über dessen der im Stirnendbereich der pilzförmig bzw. als Kugelkalotte oder konisch ausgebildet ist, das Auszugmittel 5 vorragt. Der derart vorgefertigte, zylindrische Rohkörper wird meist unter erhöhter Temperatur mit radial auf diesen einwirkenden Presswerkzeugen verdichtet, sodass er eine endgültige, zylindrische Form, wie in Fig. 14 dargestellt, erhält. Als Material für dieses Band 32 kann jedes weiche, absorbierende Material, sowie Baumwolle, Gaze oder auch mehrlagiges Material, welches eine hohe Saugfähigkeit aufweist, verwendet werden. Das Laufmetergewicht bei einer Breite die etwa der Länge 35 der Bandteile 34 entspricht, beträgt üblicherweise zwischen 10 und 35 g.

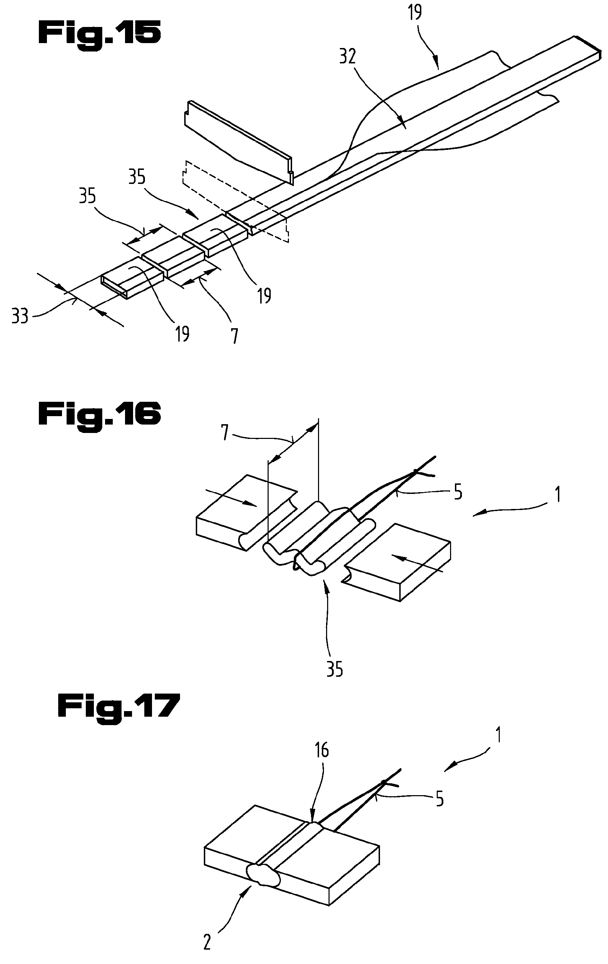

Eine weitere mögliche Ausführungsform für den Tampon 1 ist in den Fig. 15 bis 17 dargestellt. Diese spezielle Form des Tampon 1, die der sogenannten Kategorie "Teebeuteltampon" zuzuordnen ist, besteht aus einem rechteckförmigen Zuschnitt, aus einem Streifen eines absorbierenden Materials, wie Baumwolle, Gaze, Watte oder dergleichen.

Wie schematisch in Fig. 15 angedeutet, wird dieses Band 32 im Zuge des Fertigungsprozesses mit einer Materiallage, beispielsweise einer Nonwoven, zur Herstellung einer Umhüllung 19 umwickelt. Das mit der Umhüllung 19 umwickelte Band 32 wird mittels einer schematisch angedeuteten Messers in Bandteile 34 unterteilt. Diese Bandteile 34 weisen hierbei üblicherweise ein in Längsrichtung des Bandes 32 eine Länge von 20 bis 50 mm, bevorzugt 40 mm und eine Breite von 40 mm bzw. 20 bis 40 mm auf. An diesen Bandteil 34 wird in Richtung der Längsmittelachse ein Auszugmittel 5, beispielsweise ein Rückziehfaden, angenäht oder angeklebt bzw. mit dem Bandteil verwoben. Dieser ragt über eine der beiden Längsstirnseiten des Bandteiles 34 in Längsrichtung hinaus, wie dies in Fig. 16 gezeigt ist.

Das Laufmetergewicht des Bandes 32, welches zur Herstellung des Tampons verwendet wird, beträgt bei einer Breite 33 des Bandes 32 ein Laufmetergewicht zwischen 10 und 40 g, bevorzugt zwischen 10 und 25 g.

Dieser Bandteil 34 wird dann, wie in Fig. 16 gezeigt, mit zwei in Pfeilrichtung zustellbaren Pressstempeln zick-zackförmig bzw. W-förmig zusammengefaltet und, wie in Fig. 17 gezeigt, in eine etwa zylinderförmige Form verformt. In dieser verformten und verdichteten Form wird der Saugkörper 16 der aus dem Bandteil 34 hergestellt wurde, üblicherweise in einen rohrförmigen Applikator zum Einbringen in die Vagina eingeschoben.

Derartige sogenannte Teebeuteltampon (Tampon mit aufgenähtem Faden) werden üblicherweise nur mit Applikatoren in die Vagina eingebracht.

Alle Angaben bezüglich der Saugfähigkeit und der Absorptionsfähigkeit des Saugkörpers bzw. der Flüssigkeitsmengen, die mit erfindungsgemäßen Tampons aufgenommen werden können, basieren auf den Standarttest der EDANA (European Disposables and Nonwoven Association), nach der Syngina-Methode.

Die diesbezügliche EDANA-Vorschrift trägt die Bezeichnung "Tamponsabsorbency" und die Referenznummer 350.0-02. In der englischen Fassung vom Februar 2002.

Somit kann auch diese Tamponart als Tampon 1 hergestellt werden und kann der Tampon 1 in der für die Benutzerin gewohnten Weise jedoch mit Applikatoren mit geringeren Durchmesser in die Vagina mechanisch eingesetzt werden.

Des Weiteren kann der Tampon 1 sowohl als "Digitaltampon", bei welchen er von der Benutzerin manuell mit dem Finger in die Vagina eingeführt wird, als auch für Applikatortampons verwendet werden.

### Bezugszeichenaufstellung

- 1: Alltagstampon
- 2: Distales Ende
- 3: Mittelteil
- 4: Proximales Ende
- 5: Auszugmittel
- 6: Durchmesser
- 7: Länge
- 8: Rillen
- 9: Vertiefungen
- 10: Längsmittelachse
- 11: Bereich
- 12: Entfernung
- 13: Bereich
- 14: Oberfläche
- 15: Zentralbereich
- 16: Saugkörper
- 17: Faden
- 18: Kernbereich
- 19: Umhüllung
- 20: Öffnung
- 21: Durchmesser
- 22: Tiefe
- 23: Größe
- 24: Linie
- 25: Ausnehmung
- 26: Endbereich
- 27: Pfeil
- 28: Rippe
- 29: Endbereich
- 30: Endbereich
- 31: Hohlraum
- 32: Band
- 33: Breite
- 34: Bandteil
- 35: Länge
- 36: Material
- 37: Hüllkörper
- 38: Vertiefung

## Patentansprüche

1. Tampon (1) für Zwischenmenstruationstage der zumindest einen Saugkörper (16), von dem zumindest ein Teil seines Volumens aus einem saugfähigen Material (36) gebildet ist und einen Mittelteil (3), ein proximales Ende (2) und ein distales Ende (4) aufweist und einem mit dem Tampon (1) verbundenen Auszugmittel (5), wobei der Saugkörper (16) über sein gesamtes Volumen aus einem saugfähigen Material gebildet ist, **dadurch gekennzeichnet, dass** der Tampon (1) für die Tage zwischen den Menstruationsperioden mit einem Volumen des saugfähigen Materials (36) zur Aufnahme einer Flüssigkeitsmenge zwischen 0,5 g und 4 g, bevorzugt 3,5 g, ausgebildet ist und der Saugkörper (16) eine Länge kleiner 40 mm, bevorzugt eine Länge zwischen 40 und 10 mm oder zwischen 38 und 30 mm aufweist.

2. Tampon (1) nach Anspruch 1, **dadurch gekennzeichnet, dass** das saugfähige Material (36) aus faserförmigen Material gebildet ist.

3. Tampon (1) nach einem oder mehreren vorhergehenden Ansprüche, **dadurch gekennzeichnet, dass** das saugfähige Material (36) aus einem komprimierten, faserförmigen Material gebildet ist.

4. Tampon (1) nach einem oder mehreren der vorhergehenden Ansprüche, **dadurch gekennzeichnet, dass** ein Durchmesser (6) des Saugkörpers (16) bzw. eines den Saugkörper (16) umschließenden Hüllkörpers maximal 10 mm beträgt, bevorzugt einen Wert zwischen 2 und 10 mm aufweist.

5. Tampon (1) nach einem oder mehreren der vorhergehenden Ansprüche, **dadurch gekennzeichnet, dass** zumindest der Mittelteil (3) des Saugkörpers (16) oder ein diesen umhüllender Hüllkörper (19) zylinderförmig ausgebildet ist.

6. Tampon (1) nach einem oder mehreren der vorhergehenden Ansprüche, **dadurch gekennzeichnet, dass** zumindest der Mittelteil (3) des Saugkörpers (16) oder ein diesen umhüllender Hüllkörper (19) kegel- bzw. kegelstumpfförmig ausgebildet ist.

7. Tampon (1) nach einem oder mehreren der vorhergehenden Ansprüche, **dadurch gekennzeichnet, dass** sich der Zylinder oder Kegel bzw. der Kegelstumpf vom proximalen Ende (2) bis zum distalen Ende (4) durchgehend erstreckt.

8. Tampon (1) nach einem oder mehreren der vorhergehenden Ansprüche, **dadurch gekennzeichnet, dass** zumindest innerhalb des Mittelteils (3) des Saugkörpers (16) ein Kernbereich (18) aus faserförmigem Material angeordnet ist, welches bei gleichem Volumen zur Aufnahme einer geringeren Flüssigkeitsmenge ausgebildet ist als das Material (36) des Saugkörpers (16)

9. Tampon (1) nach einem oder mehreren der vorhergehenden Ansprüche, **dadurch gekennzeichnet, dass** zumindest innerhalb des Mittelteils (3) des Saugkörpers (16) ein Kernbereich (18) aus einem Material angeordnet ist, welches bei gleichem Volumen zur Aufnahme einer geringeren Flüssigkeitsmenge ausgebildet ist als das Material (36) des Saugkörpers (16) oder feuchtigkeitsabweisend ist.

10. Tampon (1) nach einem oder mehreren der vorhergehenden Ansprüche, **dadurch gekennzeichnet, dass** das faserförmige Material (36) des Saugkörpers (16) ein Gesamtgewicht aufweist welches zwischen maximal 2 g, bevorzugt 1,7 g und minimal von 0,5 g bevorzugt 0,2 g beträgt.

11. Tampon (1) nach einem oder mehreren der vorhergehenden Ansprüche, **dadurch gekennzeichnet, dass** der Saugkörper (16) mit einem Hohlraum (39) ausgebildet ist, und im Bereich seines distalen Endes (4) eine Öffnung (40) zu diesem Hohlraum (39) aufweist.

12. Tampon (1) nach einem oder mehreren der vorhergehenden Ansprüche, **dadurch gekennzeichnet, dass** das Auszugmittel (5) mit dem Saugkörper (16) und/oder mit dem Zentral- bzw. Kernbereich (15, 18) des Tampons (1) verbunden ist.

13. Tampon (1) nach einem oder mehreren der vorhergehenden Ansprüche, **dadurch gekennzeichnet, dass** eine äußere Oberfläche des Saugkörpers (16) bzw. der Mittelteil (3) zumindest zum Teil mit einer Umhüllung (19), zum Beispiel einem Nonwoven, versehen ist.

14. Tampon (1) nach einem oder mehreren der vorhergehenden Ansprüche, **dadurch gekennzeichnet, dass** das proximale Ende (2) und der Mittelteil (3) mit einer zumindest teilweise flüssigkeitsdurchlässigen Umhüllung (19), zum Beispiel einem Nonwoven, ausgebildet sind.

15. Tampon (1) nach einem oder mehreren der vorhergehenden Ansprüche, **dadurch gekennzeichnet, dass** die Umhüllung (19) durch ein Netz oder eine Lochfolie gebildet ist.

16. Tampon (1) nach einem oder mehreren der vorhergehenden Ansprüche, **dadurch gekennzeichnet, dass** das saugfähige, faserförmige Material (36) aus einem oder mehreren von Materialien wie Rayon, Baumwolle, Zellstoff, Zellstoffwatte, Tissue-Schichtstoffe, Torfmull, Bambus oder chemisch verstärkte, modifizierte oder vernetzte Zellulosefasern, gebildet ist.

17. Tampon (1) nach einem oder mehreren der vorhergehenden Ansprüche, **dadurch gekennzeichnet, dass** das saugfähige, faserförmige Material (36) durch eines oder mehrere nachstehend, synthetischen Materialien, wie Polyesterfasern, Polyolefinfasern, saugfähige Schaumstoffe, saugfähige Schwämme, saugfähige Polymere, kapillare Kanalfasern, synthetische Fasern, überwiegend offenzelligen Polyurethan-Weichschaum oder Fasern bzw. Fäden aus Rayon oder einem Strukturtyp der kristallen Modifikation von Zellulose II gebildet ist.

18. Tampon (1) nach einem oder mehreren der vorhergehenden Ansprüche, **dadurch gekennzeichnet, dass** der Kernbereich (18) des Saugkörpers (16) innerhalb eines Hüllkreises bzw. Hüllzylinders mit einem über seine Länge bevorzugt gleichen Durchmesser (21) ausgebildet ist, der bevorzugt kleiner 4 mm ausgebildet bzw. zwischen 4 mm und 1 mm beträgt.

19. Tampon (1) nach einem oder mehreren der vorhergehenden Ansprüche, **dadurch gekennzeichnet, dass** im Kernbereich (18) des Saugkörpers (16) das saugfähige, faserförmige Material (36) höher verdichtet ist als in den übrigen Bereichen des Saugköpers (16).

20. Tampon (1) nach einem oder mehreren der vorhergehenden Ansprüche, **dadurch gekennzeichnet, dass** das proximale Ende (2) rund oder abgerundet ausgebildet ist.

21. Tampon (1) nach einem oder mehreren der vorhergehenden Ansprüche, **dadurch gekennzeichnet, dass** das proximale Ende (2) mit einen sich in Richtung des distalen Endes (4) verjüngenden Konus ausgebildet ist.

22. Tampon (1) nach einem oder mehreren der vorhergehenden Ansprüche, **dadurch gekennzeichnet, dass** der Saugkörper (16) mit in Längsrichtung desselben verlaufenden Vertiefungen (9) bzw. Rillen (8) versehen ist.

23. Tampon (1) nach einem oder mehreren der vorhergehenden Ansprüche, **dadurch gekennzeichnet, dass** die Vertiefungen bzw. Rillen (9, 8) in Richtung der Längsmittelachse (10) des Saugkörpers (16) wellenförmig, bevorzugt mit gleichbleibender Höhe der Amplitude verlaufen.

24. Tampon (1) nach einem oder mehreren der vorhergehenden Ansprüche, **dadurch gekennzeichnet, dass** die Rillen (8) bzw. Vertiefungen (9) spiralförmig oder schraubenlinienförmig verlaufen.

25. Tampon (1) nach einem oder mehreren der vorhergehenden Ansprüche, **dadurch gekennzeichnet, dass** mehrere Rillen (8) bzw. Vertiefungen (9) in Umfangsrichtung über den Saugkörper (16) verteilt angeordnet sind.

## Claims

1. Tampon (1) for days between menstruation, comprising at least an absorbent body (16), of which at least a part of its volume is made from an absorbent material (36) and having a middle part (3), a proximal end (2) and a distal end (4), and an extraction means (5) connected to the tampon (1), wherein the absorbent body (16) is formed from an absorbent material throughout its entire volume, **characterized in that** the tampon (1) is designed for the days between menstruation periods having a volume of absorbent material (36) to absorb a liquid quantity of between 0.5 g and 4 g, preferably 3.5 g, and the absorbent body (16) has a length of less than 40 mm, preferably a length of between 40 and 10 mm or between 38 and 30 mm.

2. Tampon (1) according to claim 1, **characterized in that** the absorbent material (36) is formed from fibrous material.

3. Tampon (1) according to one or more of the preceding claims, **characterized in that** the absorbent material (36) is formed from a compressed, fibrous material.

4. Tampon (1) according to one or more of the preceding claims, **characterized in that** a diameter (6) of the absorbent body (16) or an enclosing body surrounding the absorbent body (16) is at most 10 mm, preferably having a value of between 2 and 10 mm.

5. Tampon (1) according to one or more of the preceding claims, **characterized in that** at least the middle part (3) of the absorbent body (16) or enclosing body (19) surrounding it is configured to be cylindrical.

6. Tampon (1) according to one or more of the preceding claims, **characterized in that** at least the middle part (3) of the absorbent body (16) or an enclosing body (19) surrounding it is configured to be conical or frustoconical.

7. Tampon (1) according to one or more of the preceding claims, **characterized in that** the cylinder or cone or truncated cone extends continuously from the proximal end (2) to the distal end (4).

8. Tampon (1) according to one or more of the preceding claims, **characterized in that** a core region (18) of fibrous material is disposed at least inside the middle part (3) of the absorbent body (16) which is configured to absorb a smaller liquid quantity than the material (36) of the absorbent body (16) for the same volume.

9. Tampon (1) according to one or more of the preceding claims, **characterized in that** a core region (18) is disposed at least inside the middle part (3) of the absorbent body (16) made from a material which is configured to absorb a smaller liquid quantity than the material (36) of the absorbent body (16) for the same volume or which is moisture-repellent.

10. Tampon (1) according to one or more of the preceding claims, **characterized in that** the fibrous material (36) of the absorbent body (16) has a total weight which is between at most 2 g, preferably 1.7 g and at least 0.5 g preferably 0.2 g.

11. Tampon (1) according to one or more of the preceding claims, **characterized in that** the absorbent body (16) is provided with a cavity (39) and has an opening (40) to this cavity (39) in the region of its distal end (4).

12. Tampon (1) according to one or more of the preceding claims, **characterized in that** the extraction means (5) is connected to the absorbent body (16) and/or to the central or core region (15, 18) of the tampon (1).

13. Tampon (1) according to one or more of the preceding claims, **characterized in that** an outer surface of the absorbent body (16) or the middle part (3) is at least partially provided with a casing (19), for example a nonwoven.

14. Tampon (1) according to one or more of the preceding claims, **characterized in that** the proximal end (2) and the middle part (3) are provided with an at least partially liquid-permeable casing (19), for example a nonwoven.

15. Tampon (1) according to one or more of the preceding claims, **characterized in that** the casing (19) is provided in the form of a net or a perforated film.

16. Tampon (1) according to one or more of the preceding claims, **characterized in that** the absorbent, fibrous material (36) is formed from one or more materials such as rayon, cotton, cellulose, cellulose wadding, layered tissue, sphagnum, bamboo or chemically reinforced, modified or cross-linked cellulose fibres.

17. Tampon (1) according to one or more of the preceding claims, **characterized in that** the absorbent, fibrous material (36) is formed from one or more of the following synthetic materials, such as polyester fibres, polyolefin fibres, absorbent foams, absorbent sponges, absorbent polymers, capillary channel fibres, synthetic fibres, predominantly open-celled flexible polyurethane foam or fibres or filaments of rayon or a structure type of the crystal modification of cellulose II.

18. Tampon (1) according to one or more of the preceding claims, **characterized in that** the core region (18) of the absorbent body (16) is configured with a preferably constant diameter (21) inside an enclosing circle or enclosing cylinder over its length which is preferably less than 4 mm or between 4 mm and 1 mm.

19. Tampon (1) according to one or more of the preceding claims, **characterized in that** the absorbent, fibrous material (36) in the core region (18) of the absorbent body (16) is compressed to a greater degree than in the other regions of the absorbent body (16).

20. Tampon (1) according to one or more of the preceding claims, **characterized in that** the proximal end (2) is configured to be round or rounded.

21. Tampon (1) according to one or more of the preceding claims, **characterized in that** the proximal end (2) is configured with a tapering cone in the direction of the distal end (4).

22. Tampon (1) according to one or more of the preceding claims, **characterized in that** the absorbent body (16) is provided with recesses (9) or grooves (8) extending in the longitudinal direction thereof.

23. Tampon (1) according to one or more of the preceding claims, **characterized in that** the recesses or grooves (9, 8) extend in the direction of the longitudinal mid-axis (10) of the absorbent body (16) in a wave-shape, preferably with a constant height of amplitude.

24. Tampon (1) according to one or more of the preceding claims, **characterized in that** the grooves (8) or recesses (9) extend in a spiral shape or helically.

25. Tampon (1) according to one or more of the preceding claims, **characterized in that** a plurality of grooves (8) or recesses (9) are distributed over the absorbent body (16) in the circumferential direction.

## Revendications

1. Tampon (1) pour des périodes intermenstruelles qui comprend au moins un corps absorbant (16) dont au moins une partie de son volume est formée d'un matériau absorbant (36) et comporte une partie centrale (3), une extrémité proximale (2) et une extrémité distale (4), et un moyen d'extraction (5) relié au tampon (1), le corps absorbant (16) étant formé sur son volume entier en un matériau absorbant, **caractérisé en ce que** le tampon (1) est formé pour les jours entre les périodes menstruelles avec un volume du matériau absorbant (36) pour absorber une quantité de liquide entre 0,5 g et 4 g, de préférence 3,5 g, et que le corps absorbant (16) présente une longueur inférieure à 40 mm, de préférence une longueur entre 40 et 10 mm ou entre 38 et 30 mm.

2. Tampon (1) selon la revendication 1, **caractérisé en ce que** le matériau absorbant (36) est formé en un matériau en forme de fibres.

3. Tampon (1) selon l'une ou plusieurs des revendications précédentes, **caractérisé en ce que** le matériau absorbant (36) est formé en un matériau comprimé en forme de fibres.

4. Tampon (1) selon l'une ou plusieurs des revendications précédentes, **caractérisé en ce qu'**un diamètre (6) du corps absorbant (16) ou d'un corps enveloppant enfermant le corps absorbant (16) est de 10 mm maximum et présente de préférence une valeur entre 2 et 10 mm.

5. Tampon (1) selon l'une ou plusieurs des revendications précédentes, **caractérisé en ce qu'**au moins la partie centrale (3) du corps absorbant (16) ou un corps enveloppant (19) enfermant celui-ci est de forme cylindrique.

6. Tampon (1) selon l'une ou plusieurs des revendications précédentes, **caractérisé en ce qu'**au moins la partie centrale (3) du corps absorbant (16) ou un corps enveloppant (19) enfermant celui-ci est de forme conique ou tronconique.

7. Tampon (1) selon l'une ou plusieurs des revendications précédentes, **caractérisé en ce que** le cylindre ou le cône ou le tronc de cône s'étend de manière continue de l'extrémité proximale (2) jusqu'à l'extrémité distale (4).

8. Tampon (1) selon l'une ou plusieurs des revendications précédentes, **caractérisé en ce qu'**au moins à l'intérieur de la partie centrale (3) du corps absorbant (16), est disposée une zone centrale (18) en un matériau en forme de fibres qui est formée, à volume égal, pour l'absorption d'une quantité de liquide moindre que le matériau (36) du corps absorbant (16).

9. Tampon (1) selon l'une ou plusieurs des revendications précédentes, **caractérisé en ce qu'**au moins à l'intérieur de la partie centrale (3) du corps absorbant (16), est disposée une zone centrale (18) en un matériau qui est formée, à volume égal, pour l'absorption d'une quantité de liquide moindre que le matériau (36) du corps absorbant (16) ou qui est hydrofuge.

10. Tampon (1) selon l'une ou plusieurs des revendications précédentes, **caractérisé en ce que** le matériau en forme de fibres (36) du corps absorbant (16) présente un poids total qui est entre 2 g maximum, de préférence 1,7 g, et 0,5 g minimum, de préférence 0,2 g.

11. Tampon (1) selon l'une ou plusieurs des revendications précédentes, **caractérisé en ce que** le corps absorbant (16) est formé avec une cavité (39) et comprend, dans la zone de son extrémité distale (4), une ouverture (40) vers cette cavité (39).

12. Tampon (1) selon l'une ou plusieurs des revendications précédentes, **caractérisé en ce que** le moyen d'extraction (5) est relié au corps absorbant (16) et/ou à la zone centrale ou de noyau (15, 18) du tampon (1).

13. Tampon (1) selon l'une ou plusieurs des revendications précédentes, **caractérisé en ce qu'**une surface extérieure du corps absorbant (16) ou la partie centrale (3) est pourvue d'une enveloppe (19), par exemple un non tissé.

14. Tampon (1) selon l'une ou plusieurs des revendications précédentes, **caractérisé en ce que** l'extrémité proximale (2) et la partie centrale (3) sont pourvues d'une enveloppe (19) au moins partiellement perméable aux liquides, par exemple d'un non tissé.

15. Tampon (1) selon l'une ou plusieurs des revendications précédentes, **caractérisé en ce que** l'enveloppe (19) est formée par un filet ou un film perforé.

16. Tampon (1) selon l'une ou plusieurs des revendications précédentes, **caractérisé en ce que** le matériau absorbant en forme de fibres (36) est formé en un ou plusieurs matériaux tels que rayon, coton, cellulose, ouate de cellulose, matériaux laminés tissus, tourbe, bambou ou des fibres de cellulose renforcées, modifiées ou réticulées.

17. Tampon (1) selon l'une ou plusieurs des revendications précédentes, **caractérisé en ce que** le matériau absorbant en forme de fibres (36) est formé en un ou plusieurs des matériaux synthétiques ci-après tels que des fibres polyesters, des fibres polyoléfines, des mousses absorbantes, des éponges absorbantes, des polymères absorbants, des fibres à canal capillaire, des fibres synthétiques, de la mousse polyuréthanne essentiellement à structure alvéolaire ouverte ou des fibres ou fils en rayon ou en un type de structure de la modification cristalline de cellulose II.

18. Tampon (1) selon l'une ou plusieurs des revendications précédentes, **caractérisé en ce que** la partie de noyau (18) du corps absorbant (16) est formée à l'intérieur d'un cercle d'enveloppe ou d'un cylindre d'enveloppe ayant un diamètre de préférence constant (21) sur sa longueur, qui est de préférence inférieur à 4 mm et qui est entre 4 mm et 1 mm.

19. Tampon (1) selon l'une ou plusieurs des revendications précédentes, **caractérisé en ce que**, dans la partie de noyau (18) du corps absorbant (16), le matériau absorbant en forme de fibres (36) est comprimé davantage que dans les autres parties du corps absorbant (16).

20. Tampon (1) selon l'une ou plusieurs des revendications précédentes, **caractérisé en ce que** l'extrémité proximale (2) est formée ronde ou arrondie.

21. Tampon (1) selon l'une ou plusieurs des revendications précédentes, **caractérisé en ce que** l'extrémité proximale (2) est formée avec un cône s'amenuisant en direction de l'extrémité distale (4).

22. Tampon (1) selon l'une ou plusieurs des revendications précédentes, **caractérisé en ce que** le corps absorbant (16) est pourvu d'évidements (9) ou de rainures (8) s'étendant dans la direction longitudinale de celui-ci.

23. Tampon (1) selon l'une ou plusieurs des revendications précédentes, **caractérisé en ce que** les évidements ou rainures (9, 8) s'étendent dans la direction de l'axe central longitudinal (10) du corps absorbant (16) sous une forme ondulée, de préférence avec un niveau constant d'amplitude.

24. Tampon (1) selon l'une ou plusieurs des revendications précédentes, **caractérisé en ce que** les rainures (8) ou évidements (9) sont en forme de spirales ou hélicoïdales.

25. Tampon (1) selon l'une ou plusieurs des revendications précédentes, **caractérisé en ce que** plusieurs rainures (8) ou évidements (9) sont répartis sur le pourtour du corps absorbant (16).
